Europäisches Patentamt

European Patent Office

Office européen des brevets

(19)

(11) Veröffentlichungsnummer: **0 205 790 B1**

(12) **EUROPÄISCHE PATENTSCHRIFT**

(45) Veröffentlichungstag der Patentschrift: **08.01.92**

(51) Int. Cl.⁵: **C12M 3/00**

(21) Anmeldenummer: **86104889.0**

(22) Anmeldetag: **10.04.86**

(54) **Träger für die Kultivierung von menschlichen und/oder tierischen Zellen in einem Fermenter.**

(30) Priorität: **18.06.85 DE 3521684**
**26.08.85 DE 3530440**

(43) Veröffentlichungstag der Anmeldung:
**30.12.86 Patentblatt 86/52**

(45) Bekanntmachung des Hinweises auf die
Patenterteilung:
**08.01.92 Patentblatt 92/02**

(84) Benannte Vertragsstaaten:
**AT BE CH DE FR GB IT LI NL SE**

(56) Entgegenhaltungen:
**EP-A- 0 046 681**
**EP-A- 0 092 302**

(73) Patentinhaber: **Anawa München Aktiengesellschaft Biologische Laboratorien
Behringstrasse 6
W-8033 Planegg(DE)**

(72) Erfinder: **Müller-Lierheim, Wolfgang Georg
Konrad, Dr.
Lichtweg 5
W-8032 Gräfelfing(DE)**
Erfinder: **Beiter, Andreas Hubertus, Dr.
Agricolastrasse 83
W-8000 München 21(DE)**

(74) Vertreter: **Nöth, Heinz, Dipl.-Phys. et al
Patentanwälte Pfenning, Meinig & Partner
Mozartstrasse 17
W-8000 München 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft einen Träger nach dem Oberbegriff des Anspruchs 1.

Um das Anwachsen von adhärenten tierischen und/oder menschlichen Zellen an der festen Fläche eines Substrats zu erreichen, ist es erforderlich, daß das Substrat eine Oberflächenbeschaffenheit aufweist, die ein gutes Anheften der Zellen ermöglicht. Es ist bekannt, daß elektrisch positiv oder negativ geladene Oberflächen als Substrat geeignet sind. Ferner sind Anheft- bzw. Wachstumsfaktoren bekannt, zum Beispiel bivalente Kationen, Fibronectin oder Serum, die auf Kunststoffoberflächen aufgebracht werden, und die das Anhaften und Wachstum der Zellen erleichtern. Die elektrische Ladung der Substratoberfläche bewirkt eine Adsorption der Anheft- bzw. Wachstumsfaktoren, an denen die Zellen anwachsen. Bestimmte Zelltypen können Fibronectin selbst synthetisieren und sind daher von sich aus in der Lage, auf geladenen Kunststoffflächen anzuwachsen, z.B. diploide Fibroblasten. Andere Zellen jedoch benötigen den Zusatz von Serum oder Fibronectin zum Kulturmedium. Der Zusatz von Serum bzw. Fibronectin als Anheftfaktor bzw. Wachstumfaktor verteuert die Kultivierung von Zellen beträchtlich und ist in vielen Fällen, beispielsweise bei der Herstellung von Therapeutika, unerwünscht.

Adhärente tierische und menschliche Zellen, insbesondere Bindegewebszellen, bilden einen Zellrasen, der nur eine Zellschicht, einen sogenannten Monolayer, zuläßt. Das bedeutet, daß die Zellen nur zweidimensional wachsen. Um hohe Zelldichten zu erreichen, die bei einer technischen Anwendung benötigt werden, ist die Verwendung großer Oberflächen erforderlich. Dies erreicht man zum Beispiel durch Verwendung kugelförmiger Trägerkörper (Microcarrier), die aus vernetztem Dextran bestehen, an dessen Oberfläche denaturiertes Collagen kovalent gebunden ist. Die schleimartigen Dextrankörper werden durch Umrühren im Kulturmedium verteilt. Durch die weiche Konsistenz der Trägerkörper soll bei Kollisionen zwischen den Trägerkörpern während des Umrührens eine Beschädigung oder Abrieb der angewachsenen Zellen vermindert werden. Wenn die bewachsenen Trägerkörper miteinander in Berührung kommen und aneinander haften, kann eine dreidimensionale Zellkultur erreicht werden (Broschüre "Microcarrier cell culture principles & methods" der Firma Pharmacia Fine Chemicals AB, Uppsala). Hierdurch läßt sich jedoch kein gezieltes dreidimensionales Zellwachstum erreichen, welches Voraussetzung ist, um Zelldichten (etwa $10^9$ Zellen/ml) zu erreichen, wie sie im menschlichen bzw. tierischen Körper vorkommen.

Bekannt ist außerdem das Hohlfasermodul, durch das das Zellkulturmedium hindurchgeschickt wird. Das Zellwachstum erfolgt in den Faserzwischenräumen. Hierbei bildet sich in Längsrichtung des Hohlfasermoduls ein Gradient der Zellversorgung mit dem Zellkulturmedium aus.

Bekannt sind außerdem poröse Keramikpatronen, durch die das Zellkulturmedium hindurchgeschickt wird. Ferner ist die Mikroverkapselung der Zellen bekannt, wobei Zellen in Na-Alginat eingekapselt werden und die Alginattröpfchen mit einem polymeren Netzwerk umschlossen werden. Das Alginat wird dann aus dem Netzwerk herausgelöst. Auch bei diesen bekannten Verfahren können Zelldichten in der Größenordnung von nur etwa $10^7$ Zellen/ml erreicht werden.

Aufgabe der Erfindung ist es daher, einen Träger der eingangs genannten Art zu schaffen, mit dem ein Zellwachstum mit Zelldichten bis annähernd den im menschlichen oder tierischen Körper vorhandenen Zelldichten von $10^9$ Zellen/ml. erreicht wird.

Bei einem Trägerkörper nach dem Oberbegriff des Anspruchs 1 wird diese Aufgabe erfindungsgemäß durch die kennzeichnenden Merkmale des Anspruchs 1 gelöst.

Bei der Erfindung bildet der Grundkörper ein gegen Druck, insbesondere Strömungsdruck, formstabiles Festbett, in welchem das Zellwachstum stattfindet. Dieses Festbett weist zwei unterschiedliche innere formstabile Zwischenraumbereiche auf, von denen die einen Zwischenräume durch die Umrißform von Perlen eines Perlpolymerisats gebildet werden können und dem dreidimensionalen Zellwachstum dienen. Die anderen kapillaren Zwischenräume des Feststoffbetts können durch Makroporen der Perlen des Polymerisats gebildet werden, wobei diese Makroporen so geringe Porendurchmesser besitzen, daß Zellen nicht hineinwachsen können, jedoch eine Möglichkeit für das Hindurchfließen des flüssigen Zellkulturmediums bieten, so daß die Versorgung der Zellen mit Medium und der Abtransport von Stoffwechselprodukten der Zellen gewährleistet bleibt. Auf diese Weise erhält man ein kontinuierliches Zellwachstum. Der durch die Erfindung geschaffene Träger kann mithin ein Baustein eines wirtschaftlich arbeitenden Gewebefermenters sein, in welchem tierische und menschliche Zell-Linien in Massenkultur gehalten werden.

Durch Ausbildung des Trägerkörpers als feste, nicht bewegte Matrix kann eine Anordnung für ein dreidimensionales Wachstum tierischer oder menschlicher Zellen geschaffen werden. Die Trägerkörper sind mit Wachstumsfaktoren beschichtet. Die Wachstumsfaktoren sind an die Grenzfläche zwischen der festen Matrix zu den Zwischenräumen gebunden, in die hinein das dreidimensionale Zellwachstum erfolgt.

Die Matrix besitzt ein Kapillarsystem (z.B. die Makroporeen des Perlpolymerisats), in das die Zellen nicht hineinwachsen können und welches aber zur Versorgung der Zellen mit Zellkulturmedium und zum Abtransport von Stoffwechselprodukten der Zellen dient. Die Matrix kann von perlförmigen Grundkörpern gebildet werden, die, wie schon erwähnt, makroporös ausgebildet sein können, wobei die Poren das Kapillarsystem bilden. Die Oberflächen der Perlen sind mit den Wachstumsfaktoren beschichtet und bilden in der Matrix die Begrenzungsflächen der Zwischenräume, in denen das dreidimensionale Zellwachstum stattfindet.

In vorteilhafter Weise kann bei der Verwendung des erfindungsgemäßen Trägers auf den Zusatz von Serum bzw. Fibronectin oder anderer Anheft- und Wachstumsfaktoren zum Kulturmedium verzichtet werden. Bei der Erfindung sind die Anheft- bzw. Wachstumsfaktoren, welche Glycoproteine, insbesondere aus Serum und hier bevorzugt aus fötalem Kälberserum, sowie Fibronectin sein können, bevorzugt über Oxiran-Gruppen kovbalent an die Oberfläche des polymeren Grundkörpers gebunden. Als perlförmiges Material für den polymeren Grundkörper eignet sich beispielsweise das im Handel erhältliche und in "forum mikrobiologie" 8 (1985) S. 296 beschriebene Perlpolymerisat.

Zur Bindung der Anheft- bzw. Wachstumsfaktoren an den Trägerkörper, insbesondere an die Flächen, welche die Zwischenräume umfassen, in denen das dreidimensionale Wachstum erfolgt, können durch andere kovalente Bindungsformen erfolgen, wie sie beispielsweise aus "Angewandte Chemie 94" (1982), Seite 838 oder "Chemiker-Zeitung", 97. Jahrgang (1973), Nr. 11, Seite 612 bekannt sind.

Durch Beladung der erfindungsgemäßen Trägerkörper, welche die Anheft- bzw. Wachstumsfaktoren an ihrer Oberfläche besitzen, mit Feederzellen, z.B. mit Makrophagen, können biologisch aktive Trägermaterialien hergestellt werden, mit deren Hilfe sich auch empfindliche Zellen in synthetischem Medium ohne Serumzusätze kultivieren lassen. Durch Verwendung entsprechender Wachstumsfaktoren kann die Zellvermehrung, aber auch der Sekundärstoffwechsel, optimiert werden.

Anhand der beiliegenden Figuren wird an einem Ausführungsbeispiel die Erfindung noch näher erläutert. Es zeigt:

Fig. 1    schematisch eine Teilansicht eines Trägers als Ausführungsbeispiel, der gebildet ist aus Perlpolymerisat und

Fig. 2    schematisch einen Gewebefermenter, in welchem der Träger aus Perlpolymerisat als Festbett in bestimmter Anordnung zum Einsatz kommt.

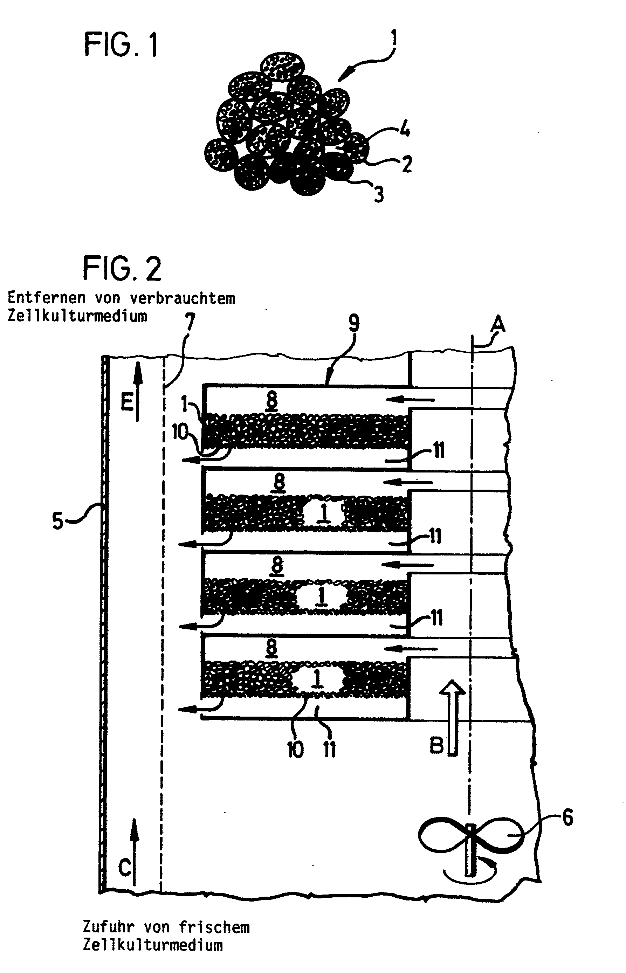

In der Fig. 1 ist in schematischer Darstellung ein kleiner Ausschnitt eines matrixförmigen Trägers 1 gezeigt, der von einem Polymerisat gebildet wird. Perlen 2 des Perlpolymerisats besitzen Durchmesser von etwa 50 bis 300 µm, insbesondere 200 µm. Die Perlen 2 besitzen Makroporen 3, deren Porendurchmesser geringer als 10 µm ist, insbesondere 0,1 bis 3,0 µm. Die Oberflächen der Perlen 2 sind mit Wachstumsfaktoren (Peptide, Proteine, Hormone etc.) für tierische oder menschliche Zellen beschichtet. Diese Oberflächen bilden Begrenzungsflächen für Zwischenräume 4, in denen das dreidimensionale Zellwachstum erfolgt. Die Makroporen 3 der Zellen bilden ein Kapillarsystem, in welches die Zellen nicht hineinwachsen können, da der Zellendurchmesser ca. 20 µm beträgt. Dieses Kapillarsystem ist durchlässig für das flüssige Wachstumsmedium, so daß eine Versorgung der Zellen mit dem Medium und der Abtransport von Stoffwechselprodukten der Zellen nicht nur während der Wachstumsphase, sondern auch in der Produktionsphase (stationäre Phase) stattfinden kann. Dieser Stoffaustausch wird durch einen bestimmten Störungsdruck erreicht, der in dem Fermenter erzeugt wird.

In der Fig. 2 ist schematisch eine Teilansicht eines Fermenters dargestellt, in welchem in Schichtform Träger 1, welche beispielsweise nach dem Ausführungsbeispiel der Fig. 1 angeordnet sind, als Bestandteil des Fermenters zum Einsatz kommen. Die Schichtdicke kann je nach zu kultivierender Zellart zwischen 0,5 und 30 mm liegen. Der Fermenter besitzt einen zylindrischen Fermentermantel 5, in desssen Achse A ein ortsfester Rührer 6 im unteren Teil des Fermenters angeordnet ist. Im Innern des Fermenters sind übereinander in ringförmigen Schichten die Träger 1 um die Achse A angeordnet. Zwischen der Anordnung der Träger 1 und dem Fermentermantel 5 befindet sich eine semipermeable Folie 7. Durch den Rührer 6, der auch durch ein Pumpwerk ersetzt sein kann, wird Zellkulturmedium entlang der Achse A in einer Strömung in Richtung eines Pfeiles B zentral und axial im Fermenter nach oben transportiert. Die Zufuhr von Zellkulturmedium in den Fermenter kann in Richtung eines Pfeiles C zwischen dem Fermentermantel 5 und der semipermeablen Folie 7 erfolgen. Die Strömungsrichtung entlang der Achse A kann auch von oben nach unten gerichtet sein.

Die in Richtung des Pfeiles B zentrale Hauptströmung des Zellkulturmediums verzweigt sich radial nach außen in die zwischen den Schichten des Trägers 1 gebildeten Zwischenräume 8. Das in die Zwischenräume 8 über den Trägern 1 befindliche Zellkulturmedium besitzt einen Strömungsdruck, der von dem durch den Rührer 6 gebildeten Pumpwerk herrührt. Aufgrund dieses Strömungsdruckes gelangt das Zellkulturmedium in die Matrix der übereinander in Schichten angeordneten Träger 1 und strömt durch dieses von oben nach unten hindurch.

Die Träger 1 sind in dem in der Fig. 2 dargestellten Rahmen 9 auf Sieben 10 angeordnet. Auf diese Weise wird gewährleistet, daß das Zellkulturmedium nach dem Hindurchtritt durch die schichtförmigen Träger 1 nach unten in Zwischenräume 11 gelangen kann. Während des Durchtritts durch die Träger 1 gibt das Zellkulturmedium Nährstoffe für das Zellwachstum ab. Auch beim Zuwachsen der Zwischenräume 4 durch dreidimensionales Zellwachstum wird ein Durchtritt des Zellkulturmediums und der Abtransport von Stoffwechselprodukten der Zellen aus den Trägern 1 gewährleistet, weil, wie schon im Zusammenhang mit der Fig. 1 erläutert, ein Kapillarsystem vorhanden ist, das beim Ausführungsbeispiel durch die Perlporen gebildet wird, in welches die Zellen nicht hineinwachsen können und welches ausreicht, um die Zellen mit weiterem frischen Zellkulturmedium, das aus den Zwischenräumen 8 kommt, zu versorgen und Stoffwechselprodukte der Zellen nach dem Durchtritt durch die Träger 1 in die Zwischenräume 11 zu transportieren. Aus den Zwischenräumen 11 gelangt das Zellkulturmedium radial nach außen. Niedermolekulare Stoffe werden durch die semipermeable Folie 7 hindurch ausgetauscht. Das "erneuerte" Medium wird nach oben in Richtung eines Pfeiles E aus dem Fermenter entfernt. Proteine als Produkte des Sekundärstoffwechsels der Zellen werden zurückgehalten und angereichert.

Das Zellkulturmedium kann in herkömmlicher Weise Aminosäuren, Kohlenstoffquellen, insbesondere Glucose und Zusätze wie Sauerstoff und Kohlensäure und gegebenenfalls Serum, enthalten. Die Wahl des Zellkulturmediums hängt von der Art der zu kultivierenden Zellen ab.

Durch die Entfernung des verbrauchten Nährmediums in Richtung des Pfeiles E wird gewährleistet, daß die Lactatkonzentration im Fermenter niedrig gehalten wird, so daß das Zellwachstum nicht beeinträchtigt wird.

Die Zufuhr von frischem Zellkulturmedium kann natürlich auch innerhalb der zylindrischen semipermeablen Folie 7 erfolgen.

## Patentansprüche

1. Träger für die Kultivierung von menschlichen und/oder tierischen Zellen in einem Fermenter mit einem polymeren Grundkörper, an desssen Oberfläche Wachstumsfaktoren für menschliche und/oder tierische Zellen kovalent gebunden sind, dadurch gekennzeichnet, daß der Grundkörper im Fermenter aus einem zu einer druckfesten Matrix geformten Feststoff mit einem für das flüssige Zellkulturmedium durchlässigen Kapillarsystem gebildet ist, in welchem kein Zellwachstum erfolgt, und mit Zwischenräumen für dreidimensionales Zellwachstum, an deren Begrenzungsflächen die Wachtumsfaktoren gebunden sind.

2. Träger nach Anspruch 1, dadurch gekennzeichnet, daß der Grundkörper ortsfest im Fermenter angeordnet ist und vom Zellkulturmedium durchströmt ist.

3. Träger nach Anspruch 1 oder 2, dadurch gekennzeichnet, daß der Grundkörper in Form einer oder mehrerer Schichten (Lamellen) im Fermenter angeordnet ist und in Richtung der Schichtdicke (senkrecht zur Lamelle) vom Zellkulturmedium durchströmt ist.

4. Träger nach einem der Ansprüche 1 oder 3, dadurch gekennzeichnet, daß der Grundkörper von einem porösen Perlpolymerisat gebildet ist.

5. Träger nach einem der Ansprüche 1 bis 4, dadurch gekennzeichnet, daß das Perlpolymerisat einen Perldurchmesser von 30 bis 1000 μm aufweist.

6. Träger nach Anspruch 5, dadurch gekennzeichnet, daß das Perlpolymerisat einen Perldurchmesser von 50 bis 300 μm aufweist.

7. Träger nach einem der Ansprüche 4 bis 6, dadurch gekennzeichnet, daß die Perlporen weniger als 10 μm Durchmesser besitzen.

8. Träger nach einem der Ansprüche 1 bis 7, dadurch gekennzeichnet, daß das den Grundkörper bildende Polymerisat derart ausgebildet ist, daß die Bindung der Wachstumsfaktoren (Peptide, Proteine, Hormone) über Oxirangruppen an die Polymerisatoberfläche erfolgt.

9. Träger nach einem der Ansprüche 1 bis 8, dadurch gekennzeichnet, daß die Wachstumsfaktoren Glycoproteine (Blutserum oder Bestandteile davon, Fibronectin, fötales Kälberserum und dgl.) sind.

10. Träger nach einem der Ansprüche 1 bis 8, dadurch gekennzeichnet, daß die Wachstumsfaktoren eine Kombination aus Stoffwechselprodukten von Zellen und auf biochemischem Weg modifizierten Naturprodukten sind.

11. Träger nach einem der Ansprüche 1 bis 10, dadurch gekennzeichnet, daß der Grundkörper auf einem Sieb im Fermenter angeordnet ist und von oben nach unten vom Zellkulturmedium durchströmt ist.

12. Träger nach Anspruch 11, dadurch gekennzeichnet, daß im hohlzylindrisch ausgebildeten Fermenter eine längs der Zylinderachse geführte zentrale Strömung des Zellkulturmediums vorhanden ist, die in radialer Richtung sich über die jeweils in horizontalen Schichten übereinander im Fermenter angeordneten Grundkörper radial nach außen verzweigt und durch den Strömungsdruck durch den Grundkörper fließt.

13. Träger nach Anspruch 12, dadurch gekennzeichnet, daß zwischen den horizontal übereinander angeordneten Schichten des Grundkörpers zwei Zwischenräume gebildet sind, von denen der eine Zwischenraum für das in radialer Richtung sich verzweigende Zellkulturmedium und der andere Zwischenraum zum radial nach außen gerichteten Führen des durch den jeweiligen Grundkörper geflossenen Zellkulturmediums dienen.

## Claims

1. A carrier for the cultivation of human and/or animal cells in a fermenter, with a polymer base body, to the surface of which growth factors for human and/or animal cells are covalently bonded, characterised in that the base body in the fermenter is formed from a solid which is formed into a pressure-resistant matrix with a capillary system which is transmissive for the liquid cell culture medium and in which no cell growth occurs, and with interstices for three-dimensional cell growth, to the boundary surfaces of which the growth factors are bonded.

2. A carrier according to claim 1 characterised in that the base body is arranged stationarily in the fermenter and has the cell culture medium flowing therethrough.

3. A carrier according to claim 1 or claim 2 characterised in that the base body is arranged in the form of one or more layers (sheets) in the fermenter and has the cell culture medium flowing therethrough in the direction of the thickness of the layers (normal to the sheet).

4. A carrier according to one of claims 1 and 3 characterised in that the base body is formed by a porous bead polymer.

5. A carrier according to one of claims 1 to 4 characterised in that the bead polymer has a bead diameter of 30 to 1000 μm.

6. A carrier according to claim 5 characterised in that the bead polymer has a bead diameter of 50 to 300 μm.

7. A carrier according to one of claims 4 to 6 characterised in that the bead pores are less than 10 μm in diameter.

8. A carrier according to one of claim 1 to 7 characterised in that the polymer forming the base body is such that bonding of the growth factors (peptides, proteins, hormones) to the polymer surface is effected by way of oxirane groups.

9. A carrier according to one of claims 1 to 8 characterised in that the growth factors are glucoproteins (blood serum or constituents thereof, fibronectin, foetal calf serum and the like).

10. A carrier according to one of claims 1 to 8 characterised in that the growth factors are a combination of cell metabolism products and biochemically modified natural products.

11. A carrier according to one of claims 1 to 10 characterised in that the base body is arranged on a sieve in the fermenter and has the cell culture medium flowing downwardly therethrough.

12. A carrier according to claim 11 characterised in that in the fermenter which is of a hollow cylindrical configuration there is a central flow of the cell culture medium which is passed along the axis of the cylinder and which in a radial direction is branched off radially outwardly over the respective base bodies arranged in horizontal layers in superposed relationship in the fermenter, and flows through the base body due to the flow pressure.

13. A carrier according to claim 12 characterised in that two intermediate spaces are formed between the layers of the base body which are arranged horizontally in superposed relationship, of which one intermediate space serves for the cell culture medium which branches off in the radial direction and the other intermediate space serves for radially outwardly directedly guiding the cell culture medium which flows through the respective base body.

## Revendications

1. Support pour la culture de cellules humaines et/ou animales dans un fermenteur avec un corps de base polymère, sur la surface duquel des facteurs de croissance pour cellules humaines et/ou animales sont fixés par des liaisons covalentes, caractérisé en ce que le corps de base dans le fermenteur se compose d'un solide, conformé en une matrice résistante à la pression, avec un système capillaire perméable au milieu de culture liquide, dans lequel aucune croissance cellulaire ne se déroule, et avec des interstices pour une croissance des cellules à trois dimensions, sur les surfaces de délimitation desquels sont liés les facteurs de croissance.

2. Support suivant la revendication 1, caractérisé en ce que le corps de base est stationnaire dans le fermenteur, et est traversé par le milieu de culture.

3. Support suivant l'une des revendications 1 et 2, caractérisé en ce que le corps de base est disposé en une ou plusieurs couches (lamelles) dans le fermenteur, et est traversé par le milieu de culture dans le sens de l'épaisseur de couche (perpendiculairement à la lamelle).

4. Support suivant l'une quelconque des revendications 1 à 3, caractérisé en ce que le corps de base se compose d'un polymérisat en perles poreux.

5. Support suivant l'une quelconque des revendications 1 à 4, caractérisé en ce que le polymérisat en perles présente un diamètre de perle de 30 à 1000 $\mu$ m.

6. Support suivant la revendication 5, caractérisé en ce que le polymérisat en perles présente un diamètre de perle de 50 à 300 $\mu$ m.

7. Support suivant l'une quelconque des revendications 4 à 6, caractérisé en ce que les pores des perles ont un diamètre de moins de 10 $\mu$m.

8. Support suivant l'une quelconque des revendications 1 à 7, caractérisé en ce que le polymérisat, formant le corps de base, a une configuration telle, que la liaison des facteurs de croissance (peptides, protéines, hormones) sur la surface du polymérisat est assurée par l'intermédiaire de groupes oxirannes.

9. Support suivant l'une quelconque des revendications 1 à 8, caractérisé en ce que les facteurs de croissance sont des glycoprotéines (sérum sanguin ou constituants de ce dernier, fibronectine, sérum foetal de veau, et autres).

10. Support suivant l'une quelconque des revendications 1 à 8, caractérisé en ce que les facteurs de croissance sont une combinaison des produits du métabolisme de cellules et de produits naturels modifiés par voie biochimique.

11. Support suivant l'une quelconque des revendications 1 à 10, caractérisé en ce que le corps de base est disposé sur un crible dans le fermenteur, et est traversé par le milieu de culture, du haut vers le bas.

12. Support suivant la revendication 11, caractérisé par un écoulement central du milieu de culture le long de l'axe du cylindre, dans le fermenteur cylindrique et creux, cet écoulement se répartissant, dans le sens radial externe, au-dessus des corps de base superposés en couches horizontales dans le fermenteur, et s'écoulant au travers du corps de base, sous l'effet de la pression d'écoulement.

13. Support suivant la revendication 12, caractérisé par deux espaces intermédiaires, formés entre les couches horizontales superposées du corps de base, l'un des espaces intermédiaires servant au milieu de culture, qui se répartit dans le sens radial, l'autre espace servant au passage, dans le sens radial externe, du milieu de culture écoulé au travers du corps de base respectif.

# FIG. 1

# FIG. 2

Entfernen von verbrauchtem
Zellkulturmedium

Zufuhr von frischem
Zellkulturmedium